(19) Europäisches Patentamt
European Patent Office
Office européen des brevets

(11) EP 0 976 818 A1

(12) EUROPÄISCHE PATENTANMELDUNG

(43) Veröffentlichungstag:
**02.02.2000 Patentblatt 2000/05**

(51) Int Cl.[7]: **C11D 3/33**, C11D 17/00, C11D 17/06, C07C 229/16

(21) Anmeldenummer: **99111836.5**

(22) Anmeldetag: **07.06.1994**

(84) Benannte Vertragsstaaten:
**AT BE CH DE DK ES FR GB IT LI NL SE**

(30) Priorität: **16.06.1993 DE 4319935**

(62) Dokumentennummer(n) der früheren Anmeldung(en) nach Art. 76 EPÜ:
**94920434.1 / 0 703 971**

(71) Anmelder: **BASF AKTIENGESELLSCHAFT**
**67056 Ludwigshafen (DE)**

(72) Erfinder:
- **Schneider, Juergen**
  **67251 Freinsheim (DE)**
- **Potthoff-Karl, Birgit**
  **67061 Ludwigshafen (DE)**
- **Kud, Alexander**
  **55234 Eppelsheim (DE)**
- **Baur, Richard**
  **67112 Mutterstadt (DE)**
- **Oftring, Alfred**
  **67098 Bad Duerkheim (DE)**
- **Greindl, Thomas**
  **94127 Neuburg (DE)**

Bemerkungen:
Diese Anmeldung ist am 19 - 06 - 1999 als Teilanmeldung zu der unter INID-Kode 62 erwähnten Anmeldung eingereicht worden.

(54) **Verwendung von Glycin-N,N-diessigsäure-Derivaten als biologisch abbaubare Komplexbildner für Erdalkali- und Schwermetallionen in pulverförmigen Kompakt- und Ultra-Kompakt-Waschmittelformulierungen und in flüssigen Waschmittelformulierungen**

(57) Verwendung von Glycin-N,N-diessigsäure-Derivaten der allgemeinen Formel I

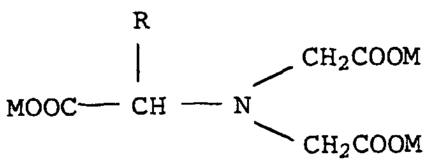

(I)

in der

R für $C_1$- bis $C_{30}$-Alkyl oder $C_2$- bis $C_{30}$-Alkenyl, welche zusätzlich als Substituenten bis zu 5 Hydroxylgruppen, Formylgruppen, $C_1$- bis $C_4$-Alkoxygruppen, Phenoxygruppen oder $C_1$- bis $C_4$-Alkoxycarbonylgruppen tragen und durch bis zu 5 nicht benachbarte Sauerstoffatome unterbrochen sein können, Alkoxylat-Gruppierungen der Formel $-(CH_2)_k-O-(A^1O)_m-(A^2O)_n-Y$, in der $A^1$ und $A^2$ unabhängig voneinander 1,2-Alkylengruppen mit 2 bis 4 C-Atomen bezeichnen, Y Wasserstoff, $C_1$- bis $C_{12}$-Alkyl, Phenyl oder $C_1$- bis $C_4$-Alkoxycarbonyl bedeutet und k für die Zahl 1, 2 oder 3 sowie m und n jeweils für Zahlen von 0 bis 50 stehen, wobei die Summe aus m + n mindestens 4 betragen muß, Phenylalkylgruppen mit 1 bis 20 C-Atomen im Alkyl, einen fünf- oder sechsgliedrigen ungesättigten oder gesättigten heterocyclischen Ring mit bis zu drei Heteroatomen aus der Gruppe Stickstoff, Sauerstoff und Schwefel, welcher zusätzlich benzanelliert sein kann, tragende $C_1$- bis $C_{20}$-Alkylgruppen, wobei alle bei den Bedeutungen für R genannten Phenylkerne und heterocyclischen Ringe noch zusätzlich als Substituenten bis zu drei $C_1$- bis $C_4$-Alkylgruppen, Hydroxylgruppen, Carboxylgruppen, Sulfogruppen oder $C_1$- bis $C_4$-Alkoxycarbonylgruppen tragen können, oder einen Rest der Formel

EP 0 976 818 A1

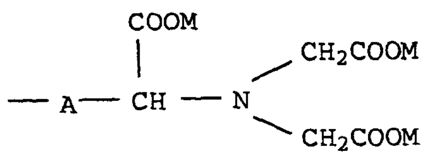

steht, wobei A eine $C_1$- bis $C_{12}$-Alkylen-Brücke, vorzugsweise eine $C_2$- bis $C_{12}$-Alkylen-Brücke, oder eine chemische Bindung bezeichnet, und

M Wasserstoff, Alkalimetall, Erdalkalimetall, Ammonium oder substituiertes Ammonium in den entsprechenden stöchiometrischen Mengen bedeutet,

als Komplexbildner für Erdalkali- und Schwermetallionen in pulverförmigen Kompakt- und Ultra-Kompakt-Waschmittelformulierungen und in flüssigen Waschmittelformulierungen.

## Beschreibung

Die vorliegende Erfindung betrifft die Verwendung von Glycin-N,N-diessigsäure-Derivaten und ihren Alkalimetall-, Erdalkalimetall-, Ammonium- und substituierten Ammoniumsalzen als Komplexbildner für Erdalkali- und Schwermetallionen in pulverförmigen Kompakt- und Ultra-Kompakt-Waschmittelformulierungen und in flüssigen Waschmittelformulierungen.

Aus den japanischen Offenlegungsschriften 80/157 695 (1) und 80/160 099 (2), zitiert in Chem. Abstr. 95 (1981), 9123 m bzw. 9124 n, ist bekannt, Alanin-N,N-diessigsäure in Form des Natriumsalzes als Builder in pulverförmig formulierten Textilwaschmitteln einzusetzen, wobei insbesondere für Baumwolltextilien eine Waschkraftverstärkung zu beobachten ist.

Die EP-A 089 136 (3) betrifft Mundhygieneprodukte, die als Calciumsequestriermittel u.a. a-Alanin-N,N-diessigsäure enthalten. Diese dienen dazu, die Menge an Calciumfluorid, welche dem Zahnschmelz zum Kariesschutz zugeführt wird, zu kontrollieren.

Als Komplexbildner für Erdalkali- und Schwermetallionen auf den verschiedensten technischen Gebieten mit ihren teilweise stark voneinander abweichenden Anforderungs- und Problemfeldern werden üblicherweise immer noch altbekannte und bewährte Systeme wie Polyphosphate, Nitrilotriessigsäure oder Ethylendiamintetraessigsäure eingesetzt. Diese Mittel zeigen allerdings gewisse Nachteile, prinzipielle Schwachpunkte sind insbesondere ihr noch verbesserungsbedürftiges Calcium- und Mangan-Bindevermögen, ihre noch nicht optimale stabilisierende Wirkung in Bleichbädern und Bleichsystemen sowie ihre meist unzureichende biologische Abbaubarkeit bzw. Eliminierbarkeit.

Aufgabe der vorliegenden Erfindung war es daher, Komplexbildner bereitzustellen, welche die Nachteile des Standes der Technik nicht mehr aufweisen.

Demgemäß wurde die Verwendung von Glycin-N,N-diessigsäure-Derivaten der allgemeinen Formel I

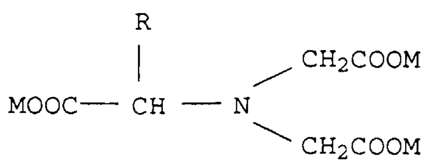

(I)

in der

R für $C_1$- bis $C_{30}$-Alkyl oder $C_2$- bis $C_{30}$-Alkenyl, welche zusätzlich als Substituenten bis zu 5 Hydroxylgruppen, Formylgruppen, $C_1$- bis $C_4$-Alkoxygruppen, Phenoxygruppen oder $C_1$-bis $C_4$-Alkoxycarbonylgruppen tragen und durch bis zu 5 nicht benachbarte Sauerstoffatome unterbrochen sein können, Alkoxylat-Gruppierungen der Formel $-(CH_2)_k-O-(A^1O)_m-(A^2O)_n-Y$, in der $A^1$ und $A^2$ unabhängig voneinander 1,2-Alkylengruppen mit 2 bis 4 C-Atomen bezeichnen, Y Wasserstoff, $C_1$- bis $C_{12}$-Alkyl, Phenyl oder $C_1$- bis $C_4$-Alkoxycarbonyl bedeutet und k für die Zahl 1, 2 oder 3 sowie m und n jeweils für Zahlen von 0 bis 50 stehen, wobei die Summe aus m + n mindestens 4 betragen muß, Phenylalkylgruppen mit 1 bis 20 C-Atomen im Alkyl, einen fünf- oder sechsgliedrigen ungesättigten oder gesättigten heterocyclischen Ring mit bis zu drei Heteroatomen aus der Gruppe Stickstoff, Sauerstoff und Schwefel, welcher zusätzlich benzanelliert sein kann, tragende $C_1$- bis $C_{20}$-Alkylgruppen, wobei alle bei den Bedeutungen für R genannten Phenylkerne und heterocyclischen Ringe noch zusätzlich als Substituenten bis zu drei $C_1$- bis $C_4$-Alkylgruppen, Hydroxylgruppen, Carboxylgruppen, Sulfogruppen oder $C_1$- bis $C_4$-Alkoxycarbonylgruppen tragen können, oder einen Rest der Formel

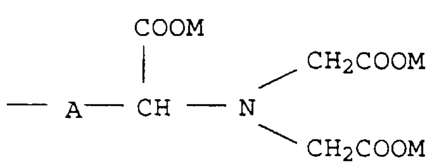

steht, wobei A eine $C_1$- bis $C_{12}$-Alkylen-Brücke, vorzugsweise eine $C_2$- bis $C_{12}$-Alkylen-Brücke, oder eine chemische Bindung bezeichnet, und

M Wasserstoff, Alkalimetall, Erdalkalimetall-, Ammonium oder substituiertes Ammonium in den entsprechenden stöchiometrischen Mengen bedeutet,

als Komplexbildner für Erdalkali- und Schwermetallionen mit Ausnahme von α-Alanin-N,N-diessigsäure als Textilwaschmittelbuilder in Pulverwaschmittelformulierungen und als Calciumsequestiermittel in Mundhygieneprodukten gefunden.

In einer bevorzugten Ausführungsform werden als Verbindungen I solche eingesetzt, bei denen R für $C_1$- bis $C_{20}$-Alkyl, $C_2$- bis $C_{20}$-Alkenyl oder einen Rest der Formel

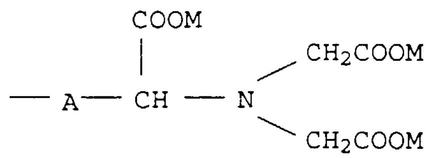

steht.

In einer besonders bevorzugten Ausführungsform werden als Verbindung I α-Alanin-N,N-diessigsäure ($R=CH_3$) und ihre Alkalimetall-, Ammonium- und substituierten Ammoniumsalze eingesetzt.

Als derartige Salze eignen sich vor allem die Natrium-, Kalium- und Ammoniumsalze, insbesondere das Trinatrium-, Trikalium- und Triammoniumsalz sowie organische Triaminsalze mit einem tertiären Stickstoffatom.

Als den organischen Aminsalzen zugrundeliegende Basen kommen insbesondere tertiäre Amine, wie Trialkylamine mit 1 bis 4 C-Atomen im Alkyl, wie Trimethyl- und Triethylamin, und Trialkanolamine mit 2 oder 3 C-Atomen im Alkanolrest, bevorzugt Triethanolamin, Tri-n-propanolamin oder Triisopropanolamin, in Betracht.

Als Erdalkalimetallsalze werden insbesondere die Calcium- und Magnesiumsalze eingesetzt.

Neben Methyl kommen für den Rest R als geradkettige oder verzweigte Alk(en)ylreste vor allem $C_2$- bis $C_{17}$-Alkyl und -Alkenyl, hierbei insbesondere geradkettige, von gesättigten oder ungesättigten Fettsäuren abgeleitete Reste, in Betracht. Beispiele für einzelne Reste R sind: Ethyl, n-Propyl, iso-Propyl, n-Butyl, iso-Butyl, sec.-Butyl, tert.-Butyl, n-Pentyl, iso-Pentyl, tert.- Pentyl, Neopentyl, n-Hexyl, n-Heptyl, 3-Heptyl (abgeleitet von 2-Ethylhexansäure), n-Octyl, iso-Octyl (abgeleitet von iso-Nonansäure), n-Nonyl, n-Decyl, n-Undecyl, n-Dodecyl, iso-Dodecyl (abgeleitet von ios-Tridecansäure), n-Tridecyl, n-Tetradecyl, n-Pentadecyl, n-Hexadecyl, n-Heptadecyl, n-Octadecyl, n-Nonadecyl, n-Eicosyl und n-Heptadecenyl (abgeleitet von Ölsäure). Es können für R auch Gemische auftreten, insbesondere solche, die sich von natürlich vorkommenden Fettsäuren und von synthetisch erzeugten technischen Säuren, beispielsweise durch Oxosynthese, ableiten.

Als $C_1$- bis $C_{12}$-Alkylen-Brücken A dienen vor allem Polymethylengruppierungen der Formel $—(CH_2)_k—$, worin k eine Zahl von 2 bis 12, insbesondere von 2 bis 8 bezeichnet, d. h. 1,2-Ethylen, 1,3-Propylen, 1,4-Butylen, Pentamethylen, Hexamethylen, Heptamethylen, Octamethylen, Nonamethylen, Decamethylen, Undecamethylen und Dodecamethylen. Hexamethylen, Octamethylen, 1,2-Ethylen und 1,4-Butylen werden hierbei besonders bevorzugt. Daneben können aber auch verzweigte $C_1$- bis $C_{12}$-Alkylengruppen auftreten, z. B. $—CH_2CH(CH_3)CH_2$-, $-CH_2C(CH_3)_2CH_2$-, $—CH_2CH(C_2H_5)—$ oder $—CH_2CH(CH_3)—$.

Die $C_1$- bis $C_{30}$-Alkyl- und $C_2$- bis $C_{30}$-Alkenylgruppen können bis zu 5, insbesondere bis zu 3 zusätzliche Substituenten der genannten Art tragen und durch bis zu 5, insbesondere bis zu 3 nicht benachbarte Sauerstoffatome unterbrochen sein. Beispiele für solche substituierten Alk(en)ylgruppen sind $—CH_2OH$, $—CH_2CH_2OH$, $—CH_2CH_2$-O-$CH_3$, $CH_2CH_2—OCH_2CH_2—O—CH_3$, $—CH_2—O—CH_2CH_3$, $—CH_2—O—CH_2CH_2—OH$, $—CH_2—CHO$, $—CH_2—OPh$, $—CH_2$-$COOCH_3$ oder $—CH_2CH_2—COOCH_3$.

Als Alkoxylat-Gruppierungen kommen insbesondere solche in Betracht, bei denen m und n jeweils für Zahlen von 0 bis 30, vor allem von 0 bis 15 stehen. $A^1$ und $A^2$ bedeuten von Butylenoxid und vor allem von Propylenoxid und von Ethylenoxid abgeleitete Gruppen. Von besonderem Interesse sind reine Ethoxylate und reine Propoxylate, aber auch Ethylenoxid-Propylenoxid-Blockstrukturen können auftreten.

Als fünf- oder sechsgliedrige ungesättigte oder gesättigte heterocyclische Ringe mit bis zu drei Heteroatomen aus der Gruppe Stickstoff, Sauerstoff und Schwefel, welche zusätzlich benzanelliert und durch die bezeichneten Reste substituiert sein können, kommen in Betracht:

Tetrahydrofuran, Furan, Tetrahydrothiophen, Thiophen, 2,5-Dimethylthiophen, Pyrrolidin, Pyrrolin, Pyrrol, Isoxazol, Oxazol, Thiazol, Pyrazol, Imidazolin, Imidazol, 1,2,3-Tri-azolidin, 1,2,3- und 1,2,4-Triazol, 1,2,3- , 1,2,4- und 1,2,5-Oxadiazol, Tetrahydropyran, Dihydropyran, 2H- und 4H-Pyran, Piperidin, 1,3- und 1,4-Dioxan, Morpholin, Pyrazan, Pyridin, α-, β- und γ-Picolin, α- und γ-Piperidon, Pyrimidin, Pyridazin, Pyrazin, 1,2,5-Oxathiazin, 1,3,5-, 1,2,3- und 1,2,4-Triazin, Benzofuran, Thionaphthen, Indolin, Indol, Isoindolin, Benzoxazol, Indazol, Benzimidazol, Chroman, Isochroman, 2H- und 4H-Chromen, Chinolin, Isochinolin, 1,2,3,4-Tetrahydroisochinolin, Cinnolin, Chinazolin, Chinoxalin, Phthalazin und Benzo-1,2,3-triazin.

N-H-Gruppierungen in den genannten heterocyclischen Ringen sollten möglichst in derivatisierter Form, etwa als N-Alkyl-Gruppierung, vorliegen.

Bei Substitution an den Phenylkernen oder den heterocyclischen Ringen treten vorzugsweise zwei (gleiche oder verschiedene) oder insbesondere ein einzelner Substituent auf.

Beispiele für gegebenenfalls substituierte Phenylalkylgruppen und heterocyclische Ringe tragende Alkylgruppen für R sind Benzyl, 2-Phenylethyl, 3-Phenylpropyl, 4-Phenylbutyl, o-, m- oder p-Hydroxylbenzyl, o-, m- oder p-Carboxylbenzyl, o-, m- oder p-Sulfobenzyl, o-, m- oder p-Methoxy oder -Ethoxycarbonylbenzyl, 2-Furylmethyl, N-Methylpiperidin-4-ylmethyl oder 2-, 3- oder 4-Pyridinylmethyl.

Bei Substitution an Phenylkernen und auch an heterocyclischen Ringen treten bevorzugt wasserlöslich machende Gruppen wie Hydroxylgruppen, Carboxylgruppen oder Sulfogruppen auf.

Als Beispiele für die genannten $C_1$- bis $C_4$-, $C_1$- bis $C_{12}$- und $C_1$- bis $C_{20}$-Alkylgruppen sind auch die entsprechenden oben aufgeführten Reste für R zu verstehen.

Eine bevorzugte Verwendung liegt in technischen Reinigungsmittelformulierungen für harte Oberflächen aus Metall, Kunststoff, Lack oder Glas.

Für die Reinigung von harten Oberflächen wurden technische Reinigungsmittelformulierungen insbesondere mit verbesserten Eigenschaften bei der Schmutzentfernung gesucht. Zur Verringerung der Abwasserbelastung ist es außerdem wünschenswert, ganz auf hierbei üblicherweise mitverwendete organische Lösungsmittel zu verzichten.

Als Einsatzgebiete für die Glycin-N,N-diessigsäure-Derivate I oder ihre Salze enthaltenden technischen Reinigungsmittelformulierungen kommen vor allem in Betracht:

- Alkalische Entroster
- Alkalische Tauchentfetter
- Allzweckreiniger
- Autowaschmittel für Bürsten- und Hochdruckwäsche
- Dampfstrahlreiniger
- Elektrolytische Entfetter, insbesondere für Stahl
- Elektrolytische Entroster
- Elektrolytische Entzunderer
- Hochalkalische Reiniger
- Hochdruckreiniger
- Kettengleitmittel für die Transportbänder von Flaschenbefüllungs- und Reinigungsanlagen
- Passivierungsmittel für Stahl
- Spritzentfetter
- Wäßrige Kaltreiniger

In der Regel enthalten diese Reinigungsmittelformulierungen 0,1 bis 30 Gew.-% Glycin-N,N-diessigsäure-Derivate I oder deren Salze.

Für die einzelnen Einsatzgebiete übliche Formulierungen sind dem Fachmann im Prinzip bekannt. In der Regel enthalten solche Formulierungen neben den Komplexbildnern 1 bis 35 Gew.-% Tenside anionischer oder vorzugsweise nichtionischer Natur, welche je nach Einsatzzwecke schäumend oder schaumarm sind, sowie gewünschtenfalls als weitere Hilfsmittel weitere Komplexbildner, Gerüststoffe, Schaumdämpfer, Emulgatoren, Korrosionsinhibitoren, Reduktionsmittel, Lösevermittler, Dispergiermittel und Konservierungsmittel in den hierfür üblichen Konzentrationen. Je nach Einsatzzweck können auch noch andere Komponenten mit spezieller Wirkung hinzukommen. Auf organische Lösungsmittel kann bei den beschriebenen Formulierungen weitgehend verzichtet werden.

Rezeptvorschläge für derartige technische Reinigungsformulierungen finden sich beispielsweise in der Technischen Information "Technische Reinigungsmittel" TI/ES 1167d vom Januar 1991 der BASF Aktiengesellschaft; die dort angegebenen Komplexbildner des Standes der Technik sind durch Glycin-N,N-diessigsäure-Derivate I bzw. ihre

Salze zu ersetzen.

Eine weitere bevorzugte Verwendung für Glycin-N,N-diessigsäure-Derivate I und ihre Salze liegt in alkalischen Reinigungsmittelformulierungen für die Getränke- und Nahrungsmittelindustrie, insbesondere für die Flaschenreinigung in der Getränkeindustrie sowie die Apparatereinigung in Molkereien, in Brauereien, in der Konserven-, der Backwaren-, der Zucker-, der fettverarbeitenden und der fleischverarbeitenden Industrie.

Für die Reinigung von Behältnissen und Apparaturen in der Getränke- und Nahrungsmittelindustrie wurden Formulierungen insbesondere mit verbesserten Eigenschaften bei der Schmutzentfernung gesucht. Zur Verringerung der Abwasserbelastung ist es außerdem wünschenswert, ganz auf organische Lösungsmittel in derartigen Formulierungen zu verzichten.

Die vorliegenden alkalischen Reinigungsmittelformulierungen weisen in der Regel pH-Werte von 8 bis 14, vorzugsweise von 9 bis 13, insbesondere von 10 bis 12, auf.

Ein bevorzugtes Einsatzgebiet für die beschriebenen Reinigungsmittelformulierungen ist die Flaschenreinigung in der Getränkeindustrie, insbesondere mit automatischen Flaschenspülmaschinen mit Stundenleistungen bis zu üblicherweise 30.000 bis 70.000 Flaschen. Die verschmutzten Flaschen enthielten beispielsweise Bier, Milch, Erfrischungsgetränke, Fruchtsäfte, Süßmost oder Mineralwasser.

Ein weiteres bevorzugtes Einsatzgebiet für die beschriebenen Reinigungsmittelformulierungen ist die Apparatereinigung in Molkereien. Bei der Reinigung von Butterfertigern, bei der es hauptsächlich auf die Entfettung ankommt, können sie mit vorteilhafter Wirkung eingesetzt werden. Insbesondere jedoch dort, wo Rückstände oder Beläge aus Calciumphosphat, anderen Calciumsalzen zumeist organischer Säuren und Casein ("Milchstein") zu entfernen sind, also z. B. bei Milch-Plattenerhitzern, Tellereinsätzen von Milchzentrifugen oder Lager- und Transport-Tanks für Milch, eignen sich Glycin-N,N-diessigsäure-Derivate I bzw. ihre Salze enthaltende Reinigungsmittel in hervorragender Weise.

Ein weiteres bevorzugtes Einsatzgebiet für die beschriebenen Reinigungsmittelformulierungen ist die Apparatereinigung in Brauereien. Hier sind vor allem Rückstände oder Beläge aus Calciumoxalat, Hopfenharzen und Eiweißverbindungen ("Bierstein") zu entfernen, beispielsweise aus Gärtanks, Lagertanks oder Bierleitungen.

Ein weiteres bevorzugtes Einsatzgebiet für die beschriebenen Reinigungsmittelformulierungen ist die Apparatereinigung in der Konservenindustrie. Beim Erhitzen der mit Nahrungsmitteln gefüllten und geschlossenen Blechdosen, üblicherweise in einem Autoklaven, oder bei der Endreinigung von Dosen, z. B. in einer Durchlaufspritzmaschine, müssen Reinigungsmittel mitverwendet werden, die die Reste des Abfüllgutes abwaschen, ohne das Weißblech oder dessen Lackierung anzugreifen. Außerdem soll das Reinigungsmittel verhindern, daß sich Wassersteinbeläge auf den Dosen oder in den Apparaten abscheiden.

Ein weiteres bevorzugtes Einsatzgebiet für die beschriebenen Reinigungsmittelformulierungen ist die Apparatereinigung in der Backwarenindustrie, insbesondere die Reinigung von Back- und Teigformen, welche mit angebrannten Backfett- und Teigresten verunreinigt sind. Die Reinigung geschieht üblicherweise durch Abkochen mit den alkalischen Reinigungslösungen oder durch Waschen in Durchlaufspritzanlagen.

Ein weiteres bevorzugtes Einsatzgebiet für die beschriebenen Reinigungsmittelformulierungen ist die Apparatereinigung in der Zuckerindustrie. Bei der Gewinnung von Saccharose aus Zuckerrüben oder Zuckerrohr fallen Calciumsalze enthaltende Verunreinigungen oder Rückstände an, für deren Entfernung sich die Glycin-N,N-diessigsäure-Derivate I bzw. ihre Salze enthaltenden beschriebenen Formulierungen in hervorragender Weise eignen.

Ein weiteres bevorzugtes Einsatzgebiet für die beschriebenen Reinigungsmittelformulierungen ist die Apparatereinigung in der fettverarbeitenden Industrie, die aus Fetten tierischen oder pflanzlichen Ursprungs vor allem Schmalz, Talg, Speiseöle oder durch katalytische Hydrierung gehärtete Fette oder fette Öle, z. B. Margarine, erzeugt. Derartige Produkte stellen neben ihrer Bedeutung auf dem Nahrungsmittelbereich auch wichtige Rohstoffe für die Herstellung von Produkten zur Textilveredlung, von Anstrichmitteln, Lederpflegemitteln, kosmetischen Präparaten, Kerzen, Seifen, Tensiden, Schmierstoffen, Weichmachern, Zement- und Asphaltzusätzen sowie von Kunststoffen dar.

Ein weiteres bevorzugtes Einsatzgebiet für die beschriebenen Reinigungsmittelformulierungen ist die Apparatereinigung in der fleischverarbeitenden Industrie. Hier müssen insbesondere wassersteinverhütende Reinigungsmittel eingesetzt werden, z. B. in den sog. Dampfstrahl-Reinigungsgeräten, bei denen ein heißes Dampf-Flüssigkeits-Gemisch auf die zu reinigenden Apparate und Geräte gestrahlt wird.

Die beschriebenen Glycin-N,N-diessigsäure-Derivate I bzw. ihre Salze enthaltenden alkalischen Reinigungsmittelformulierungen können weitgehend frei von organischen Lösungsmitteln zum Einsatz gebracht werden. Somit wird eine mögliche Umweltbelastung weitgehend ausgeschlossen.

Eine für die aufgezählten Einsatzgebiete der Getränke- und Nahrungsmittelindustrie übliche wäßrige Reinigungsmittelformulierung enthält

(i) 0,05 bis 30 Gew.-%, vorzugsweise 0,1 bis 25 Gew.-%, insbesondere 0,5 bis 15 Gew.-% Glycin-N,N-diessigsäure-Derivate I oder Alkalimetall-, Ammonium- oder substituierte Ammoniumsalze hiervon,

(ii) 2 bis 50 Gew.-%, vorzugsweise 5 bis 40 Gew.-%, insbesondere 8 bis 25 Gew.-% Alkalimetallhydroxid, -carbonat, -silicat oder einer Mischung hieraus und

(iii) 1 bis 30 Gew.-%, vorzugsweise 2 bis 25 Gew.-%, insbesondere 3 bis 20 Gew.-% Tenside.

Hierbei eignen sich als Komponente (ii) vor allem Natrium- und Kaliumhydroxid, daneben aber auch Natrium- und Kaliumcarbonat; es können auch Mischungen der genannten Alkalien eingesetzt werden.

Als Tenside (iii) können alle üblichen anionischen oder nichtionischen Tenside oder Mischungen hieraus verwendet werden, vor allem eignen sich jedoch Alkylsulfate, Alkylsulfonate, Fettalkoholalkoxylate, Oxoalkoholalkoxylate, Alkylpolyglucoside und Fettaminalkoxylate.

Diese Zusammensetzung stellt eine Grundformulierung für alle genannten Anwendungsgebiete dar. Im einzelnen innerhalb dieser Grundformulierung voneinander abweichende Zusammensetzung sind durch die verschiedenen Arten von Nahrungsmittel- und Getränkeverschmutzungen, die unterschiedlichen Mengen an Erdalkalimetallionen in diesen Rückständen und Belägen sowie durch die unterschiedlich empfindlichen Materialien der zu reinigenden Behältnissen und Apparaturen bei den verschiedenen Anwendungsgebieten zu erklären. In diesem Zusammenhang ist auch erwähnenswert, daß die beschriebenen alkalischen Reinigungsmittelformulierungen, welche Glycin-N,N-diessigsäure-Derivate I oder ihre Salze enthalten, in der Regel keine Korrosionen, auch bei empfindlichen Apparatematerialien, hervorrufen.

Die oben beschriebene Grundformulierung aus den Komponenten (i) bis (iii) kann noch übliche Hilfsmittel in den hierbei üblichen Konzentrationen enthalten, beispielsweise Desinfektionsmittel zur Erzielung des angestrebten bakteriologischen Reinheitsgrades, Netzmittel, Lösevermittler, Korrosionsinhibitoren oder Konservierungsmittel.

Eine weitere bevorzugte Verwendung für Glycin-N,N-diessigsäure-Derivate I und ihre Salze liegt in Geschirreinigungsmittelformulierungen, insbesondere in phosphatfreien Mitteln für das maschinelle Geschirreinigen in Geschirrspülmaschinen im Haushalt oder in Gewerbebetrieben, z. B. Großküchen oder Restaurants.

Eine weitere bevorzugte Verwendung für Glycin-N,N-diessigsäure-Derivate I und ihre Salze liegt in Bleichbädern in der Papierindustrie. Hier werden Komplexbildner bei der reduktiven Bleiche, z. B. mit Natriumdithionit, oder bei der oxidativen Bleiche, z. B. mit Wasserstoffperoxid, benötigt, um die Effektivität des Bleichprozesses, d. h. den Weißgrad des Holzschiffes zu erhöhen. Die Komplexbildner dienen so zur Eliminierung von Schwermetallkationen, hauptsächlich von Eisen, Kupfer und insbesondere Mangan, die auch bei der Harzleimung mit Alaun und Natriumresinat störend durch die Bildung unlöslicher Salze wirken. Die Ablagerung von Eisen auf Papier führt zu "heißen Flekken", an denen die oxidative katalytische Zerstörung der Zellulose beginnt.

Ein typische Formulierung eines derartigen wäßrigen reduktiven Bleichbades in der Papierindustrie für Holzschliff (beispielsweise 4 % Stoffdichte) enthält 0,05 bis 0,1 Gew.-% Komplexbildner I und ca. 1 Gew.-% Natriumdithionit, jeweils bezogen auf den Holzschliff. Die Badtemperatur beträgt ca. 60, die Bleichzeit normalerweise 1 Stunde und der pH-Wert ca. 5,8.

Eine typische Formulierung eines derartigen wäßrigen oxidativen Bleichbades in der Papierindustrie für Holzschliff (beispielsweise 20 % Stoffdichte) enthält 0,05 bis 0,15 Gew.-% Komplexbildner I, ca. 2 Gew.-% Wasserglas, ca. 0,75 Gew.-% NaOH und ca. 1 Gew.-% H202, jeweils bezogen auf den Holzschliff. Die Badtemperatur beträgt ca. 50°C und die Bleichzeit normalerweise 2 Stunden.

Eine weitere bevorzugte Verwendung für Glycin-N,N-diessigsäure-Derivate I und ihre Salze liegt in photographischen Bleich- und Bleichfixierbädern. In der photographischen Industrie kann man diese Verbindungen in solchen Bädern, die mit hartem Wasser angesetzt werden, verwenden, um die Ausfällung schwerlöslicher Calcium- und Magnesiumsalze zu verhindern. Die Ausfällungen führen zu Grauschleiern auf Filmen und Bildern sowie Ablagerungen in den Tanks, die somit vorteilhaft vermieden werden können. Als Eisen-IIII-Komplexbildnerlösungen können sie vorteilhaft in Bleichfixierbädern eingesetzt werden, wo sie die aus ökologischen Gründen bedenklichen Hexacyanoferratlösungen ersetzen können.

Eine typische derartige wäßrige photographische Bleich- bzw. Bleichfixierbadformulierung sieht folgendermaßen aus:

| | |
|---|---|
| Eisen(III)-Komplex mit Komplexbildner I | 0,04 bis 0,4 mol/l |
| freier Komplexbildner I | bis 1,3 mol/l |
| Natriumthiosulfat | 0,2 bis 2,0 mol/l |
| Natriumsulfit | 0,2 bis 0,3 mol/l |

Der pH-Wert eines solchen Bades liegt normalerweise bei 4 bis 8.

Eine weitere bevorzugte Verwendung für Glycin-N,N-diessigsäure-Derivate I und ihre Salze liegt in Vorbehandlungs- und Bleichbädern in der Textilindustrie. Unter Vorbehandlungsbädern sind insbesondere Entschlichtungsbäder und alkalische Vorbehandlungs- oder Mercerisierungsbäder zu verstehen. In der Textilindustrie können diese Verbindungen somit zur Entfernung von Schwermetallspuren während des Herstellungsprozesses von natürlichen und synthetischen Fasern wie Baumwolle, Wolle oder Polyester dienen. Dadurch werden viele Störungen wie Schmutzflecken und Streifen auf dem Textilgut, Verlust des Glanzes, schlechte Benetzbarkeit, unegale Färbungen und Farbfehler verhindert.

Ein typisches derartiges wäßriges Vorbehandlungsbad bei der Textilherstellung enthält:

| | |
|---|---|
| 0,1 bis 10 Gew.-% | des Komplexbildners I, |
| 0,5 bis 20 Gew.-% | üblicher Netz- oder Emulgiermittel, |
| 0 bis 10 Gew.-% | eines Reduktionsmittels wie Natriumdithionit, |
| 0 bis 5 Gew.-% | eines Puffergemisches zur Einstellung eines pH-Wertes zwischen 5 und 10 |

sowie weitere übliche Hilfsmittel wie Konservierungsmittel oder Entschlichtungsmittel, z. B. Enzyme wie Amylase.

Eine weitere bevorzugte Verwendung für Glycin-N,N-diessigsäure-Derivate I und ihre Salze liegt in galvanischen Bädern zur Maskierung von verunreinigenden Schwermetallkationen. Sie dienen hier als Ersatz für die hochtoxischen Cyanide.

Als typische Zusammensetzung eines derartigen wäßrigen galvanischen Bades zur Abscheidung von beispielsweise Kupfer, Nickel, Zink oder Gold sei das folgende Kupferbad angeführt:

| | |
|---|---|
| ca. 10 g/l | Kupfer(II) -sulfat-Pentahydrat |
| 10 bis 12 g/l | Formaldehyd |
| 12 bis 15 g/l | Komplexbildner I |
| 1 bis 2 g/l | eines $C_{13}/C_{15}$-Oxoalkohols, welcher mit 12 mol Ethylenoxid und 6 mol Propylenoxid umgesetzt wurde, als Netzmittel |

Dieses Bad wird üblicherweise mit Natronlauge auf pH 13 eingestellt; es kann noch übliche Stabilisierungsmittel wie Amine oder Natriumcyanid enthalten.

Als weitere bevorzugte Verwendung werden in der Pflanzenernährung zur Behebung von Schwermetalldefiziten Kupfer-, Eisen-, Mangan- und Zink-Komplexe der Verbindungen I eingesetzt. Die Schwermetalle werden so als Chelate zugegeben, um die Ausfällung als biologisch inaktive, unlösliche Salze zu verhindern.

Generell können Glycin-N,N-diessigsäure-Derivate I und ihre Salze in vorteilhafter Weise überall dort eingesetzt werden, wo bei technischen Verfahren Ausfällungen von Calcium-, Magnesium- und Schwermetallsalzen stören und verhindert werden sollen, beispielsweise zur Verhinderung von Ablagerungen und Verkrustungen in Kesseln, Rohrleitungen, an Sprühdüsen oder allgemein an glatten Oberflächen.

Sie können zur Stabilisierung von Phosphaten in alkalischen Entfettungsbädern und Verhinderung der Ausfällung von Kalkseifen dienen und verhindern dadurch das "Anlaufen" von Nichteisenoberflächen und verlängern die Standzeiten von alkalischen Reinigerbädern.

Die Kühlwasserbehandlung mit den Verbindungen I verhindert Ablagerungen bzw. löst bereits vorhandene wieder auf. Ein Vorteil ist die generelle Anwendbarkeit in alkalischem Medium und damit die Beseitigung von Korrosionsproblemen.

Bei der Polymerisation von Kautschuk können sie zur Herstellung der dabei verwendeten Redoxkatalysatoren verwendet werden. Sie verhindern zusätzlich das Ausfällen von Eisenhydroxid im alkalischen Polymerisationsmilieu.

In Pulverwaschmittelformulierungen für die Textilwäsche können die Verbindungen I als Komplexbildner oder als Gerüststoff (Builder) eingesetzt werden. Von $\alpha$-Alanin-N,N-diessigsäure ist eine derartige Verwendung als Builder schon bekannt. Neben den herkömmlichen Formulierungen (Schüttdichte ca. 450 g/l) gewinnen dabei immer mehr Kompakt- und Ultra-Kompaktwaschmittel (Schüttdichte $\geq$ 700 g/l) an Bedeutung. Kompaktwaschmittelformulierungen haben gegenüber herkömmlichen Pulverwaschmitteln bekanntermaßen einen höheren Gehalt an waschaktiver Substanz (Tensiden), Buildern (z.B. Zeolithen), Bleichmitteln und Polymeren. Die Verbindungen I sind in solchen Kompaktwaschmittelformulierungen üblicherweise in Mengen von 0,1 bis 25 Gew.-%, insbesondere 1 bis 15 Gew.-%, wirksam.

In flüssigen Waschmittelformulierungen für die Textilwäsche können die Verbindungen I als Komplexbildner in einer Menge von 0,05 bis 20 Gew.-%, bezogen auf das Gesamtgewicht der Waschmittelformulierung, eingesetzt werden.

In flüssigen Waschmittelformulierungen können die Verbindungen I weiterhin auch als Konservierungsmittel, zweckmäßigerweise in einer Menge von 0,05 bis 1 Gew.-%, bezogen auf das Gesamtgewicht der Waschmittelformulierung, eingesetzt werden.

In Seifen verhindern sie metallkatalysierte oxidative Zersetzungen.

Als weitere Anwendungen kommen beispielsweise Anwendungen in Pharmazeutika, Kosmetika und Nahrungsmitteln in Betracht, um z. B. die metallkatalysierte Oxidation von olefinischen Doppelbindungen und damit das Ranzigwerden der Erzeugnisse zu verhindern.

Weitere Anwendungsgebiete für die Verbindungen I sind die Rauchgaswäsche, und zwar die Entfernung von $NO_x$ aus Rauchgasen, die $H_2S$-Oxidation, die Metallextraktion sowie die Anwendung als Katalysatoren für organische Synthesen, z. B. die Luftoxidation von Paraffinen oder die Hydroformylierung von Olefinen zu Alkoholen.

Eine vorteilhafte Wirkung der Glycin-N,N-diessigsäure-Derivate I bzw. ihrer Salze liegt der Bleichmittelstabilisierung, beispielsweise bei der Bleiche von Textilien, Zellstoff oder Papierstoff. Spuren von Schwermetallen wie Eisen, Kupfer und Mangan kommen in den Komponenten des Bleichbades selbst, im Wasser und im zu bleichenden Gut vor und katalysieren die Zersetzung des Bleichmittels. Die Komplexbildner I binden diese Metallionen und verhindern die unerwünschte Zersetzung des Bleichsystems während der Lagerung und bei der Anwendung. Dadurch erhöht sich die Effizienz des Bleichsystems und Schädigungen des zu bleichenden Gutes werden zurückgedrängt.

Eine weitere vorteilhafte Wirkung der Glycin-N,N-diessigsäure-Derivate I bzw. ihrer Salze liegt in der starken bleichaktivierenden Wirkung von Komplexen der Verbindungen I mit Mangan, insbesondere Mangan der Oxidationsstufe II und IV. Solche Komplexe lassen sich als Ersatz für übliche Bleichaktivatoren in Textilwaschmittel-Formulierungen als Bleichkatalysatoren in Mengen im ppm-Bereich verwenden.

Glycin-N,N-diessigsäure-Derivate I und ihre Salze eignen sich vor allem deshalb so gut für die beschriebenen Anwendungszwecke, weil sie außerordentlich effektive Komplexbildner für Erdalkalimetallionen und für Schwermetallionen, insbesondere für Calcium und Mangan, darstellen. Ihr Calcium- und ihr Mangan-Bindevermögen sind außergewöhnlich hoch.

Weitere Vorteile sind ihr sehr geringes Toxizitätspotential und ihre gute biologische Abbaubarkeit. So zeigt α-Alanin-N,N-diessigsäure im Zahn-Wellens-Test unter Standardbedingungen eine biologische Abbaubarkeit > 90 % (28-Tage-Wert), wogegen beispielsweise Ethylendiamintetraessigsäure unter gleichen Bedingungen einen Wert von < 10 % ergibt.

Im Zusammenhang mit ihrer guten biologischen Abbaubarkeit ist es auch sehr vorteilhaft, daß die die Verbindungen I enthaltenden Reinigungsmittelformulierungen meist weitgehend frei von organischen Lösungsmitteln zum Einsatz gebracht werden können. Somit wird eine mögliche Umweltbelastung noch weitgehender ausgeschlossen.

Gegenstand der vorliegenden Erfindung ist auch ein Verfahren zur Herstellung von Glycin-N,N-diessigsäure-Derivaten I und ihren Alkalimetall-, Erdalkalimetall-, Ammonium- und substituierten Ammoniumsalzen, welches dadurch gekennzeichnet ist, daß man

A) entsprechende 2-substituierte Glycine oder 2-substituierte Glycinnitrile oder verdoppelte Glycine der Formel

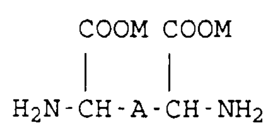

oder verdoppelte Glycinnitrile der Formel

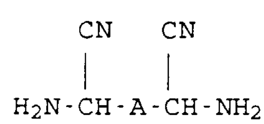

mit Formaldehyd und Cyanwasserstoff oder Alkalimetallcyanid oder

B) Iminodiessigsäure oder Iminodiacetonitril mit entsprechenden Monoaldehyden oder Dialdehyden der Formel OHC-A-CHO und Cyanwasserstoff oder Alkalimetallcyanid

umsetzt und anschließend noch vorhandene Nitrilgruppen zu Carboxylgruppe hydrolysiert.

Die beiden geannten Ausführungsformen A und B stellen Beispiele für die "Strecker-Synthese" dar, bei der allgemein Aldehyde mit Ammoniak oder Aminen und Blausäure ("saure" Variante) oder Cyaniden ("alikalische" Variante) zu Aminosäuren oder Derivate hiervon umgesetzt werden.

Die "alkalische" Variante der Strecker-Synthese wird beispielsweise in der US-A 3 733 355 (4) in allgemeiner

Form dargestellt. Die dort aufgeführten Beispiele zeigen jedoch, daß immer ein hoher Anteil an Nebenprodukten, vor allem an unerwünschter Glykolsäure, auftritt; dies läßt sich aus den Umsätzen von nur maximal ca. 89 % schließen.

Die "saure" Variante der Strecker-Synthese ist beispielsweise aus der DE-A 20 27 972 (5) bekannt. Dort wird die Herstellung von Carboxymethyliminodiacetonitril ausgehend von Glycin, Formaldehyd und Blausäure in saurem Medium beschrieben. Hierbei wird empfohlen, zusätzliche Säure zuzugeben, um den pH-Wert im Bereich unterhalb von 7 zu halten.

Aufgabe der vorliegenden Erfindung war es auch, ein effektiveres und wirtschaftlicheres Verfahren zur Herstellung von Glycin-N,N-diessigsäure-Derivaten I bereitzustellen, das insbesondere die Bildung von unerwünschten Nebenprodukten unterdrückt und auf zusätzliche Hilfsstoffe, beispielsweise zur pH-Wert-Regulierung, verzichten kann.

Demgemäß wurde das oben definierte Verfahren gefunden.

Als besonders vorteilhaft haben sich die Varianten herausgestellt, bei denen mit Cyanwasserstoff ("saure" Variante) gearbeitet wird. Zweckmäßigerweise setzt man wasserfreien Cyanwasserstoff ein, der großtechnisch üblicherweise in dieser Form gehandhabt wird. Ganz besonders vorteilhaft sind hierbei Umsetzungen, die von 2-substituierten Glycinen oder verdoppelten Glycinen der Formel

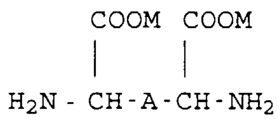

oder von Iminodiacetonitril ausgehen.

Die Umsetzung gemäß A oder B wird bevorzugt in Wasser, aber auch in einem organischen Lösungsmittel oder in Mischungen hieraus durchgeführt. Als organische Lösungsmittel kommen vorzugsweise solche zum Einsatz, die mit Wasser teilweise oder vollständig mischbar sind, z. B. Methanol, Ethanol, n-Propanol, iso-Propanol, tert.-Butanol, Dioxan oder Tetrahydrofuran. Auch Lösungsvermittler können verwendet werden.

Man setzt bei Ausführungsform A zweckmäßigerweise pro Mol Aminoverbindung 2 bis 2,6 mol Formaldehyd, vorzugsweise in Form seiner wäßrigen ca. 30 gew.-%igen Lösung, bzw. 2 bis 2,6 mol Aldehyd, in wasserfreier Form oder als wäßrige Lösung, und 2 bis 2,3 mol Cyanwasserstoff oder Alkalimetallcyanid, beispielsweise Natrium- oder Kaliumcyanid, ein. Die Umsetzung wird normalerweise im Falle von wasserfreiem Cyanwasserstoff bei Temperaturen von 0 bis 120°C, insbesondere 15 bis 80°C, und im Falle von Alkalimetallcyaniden bei 40 bis 110°C, insbesondere 70 bis 100°C, vorgenommen. Für die Umsetzung mit wasserfreiem Cyanwasserstoff kommt bei Mitverwendung von Mineralsäuren wie Schwefel-, Salz- oder ortho-Phosphorsäure bei Ausführungsform B in der Regel ein pH-Bereich von 0 bis 11, insbesondere von 1 bis 9, in Betracht, bei der Umsetzung mit Alkalimetallcyaniden arbeitet man üblicherweise bei pH 10 bis 14, insbesondere 11 bis 13.

An diese Umsetzung schließt sich eine Hydrolyse noch vorhandener Nitrilgruppen zu Carboxylgruppen an, welche in an sich bekannter Weise in wäßrigem Reaktionsmedium in Gegenwart von Basen wie Natron- oder Kalilauge oder von Säuren wie Schwefel- oder Salzsäure bei Temperaturen von 20 bis 110°C, insbesondere 40 bis 100°C, durchgeführt wird.

Die als Ausgangs-Aminoverbindungen verwendeten Glycin- und Glycinnitril-Derivate können sowohl als Racemate als auch als enantiomerenreine D- oder L-Verbindungen eingesetzt werden.

Entsprechend den Reaktionsbedingungen erhält man die Glycin-N,N-diessigsäure-Derivate I als freie Carbonsäure oder beispielsweise als Alkalimetallsalz. Aus der freien Säure können durch Neutralisation mit den entsprechenden Basen, beispielsweise Aminbasen, die gewünschten Salze ohne Schwierigkeit hergestellt werden.

Die Glycin-N,N-diessigsäure-Derivate I und ihre Salze lassen sich aus ihren Lösungen problemlos in reiner Form isolieren. Hierfür bieten sich insbesondere Sprüh- oder Gefriertrocknung, Kristallisation und Fällung an. Es kann vorteilhaft sein, die bei der Herstellung angefallene Lösung direkt einer technischen Verwendung zuzuführen.

Gegenstand der vorliegenden Erfindung sind auch die in der Literatur noch nicht bekannten in 2-Position durch den Rest R substituierten Glycin-N,N-diacetonitrile und Glycinnitril-N,N-diacetonitrile, bei denen R für $C_1$- bis $C_{30}$-Alkyl oder $C_2$- bis $C_{30}$-Alkenyl, welche zusätzlich als Substituenten bis zu 5 Hydroxylgruppen, Formylgruppen, $C_1$- bis $C_4$-Alkoxygruppen, Phenoxygruppen oder C1- bis C4-Alkoxycarbonylgruppen tragen und durch bis zu 5 nicht benachbarte Sauerstoffatome unterbrochen sein können, Alkoxylat-Gruppierungen der Formel $-(CH_2)_k-O-(A^1O)_m-(A^2O)_n-Y$, in der $A^1$ und $A^2$ unabhängig voneinander 1,2-Alkylengruppen mit 2 bis 4 C-Atomen bezeichnen, Y Wasserstoff, $C_1$- bis $C_{12}$-Alkyl, Phenyl oder $C_1$- bis $C_4$-Alkoxycarbonyl bedeutet und k für die Zahl 1, 2 oder 3 sowie m und n jeweils für Zahlen von 0 bis 50 stehen, wobei die Summe aus m + n mindestens 4 betragen muß, Phenylalkylgruppen mit 1 bis 20 C-Atomen im Alkyl, einen fünf- oder sechsgliedrigen ungesättigten oder gesättigten heterocyclischen Ring mit bis zu drei Heteroatomen aus der Gruppe Stickstoff, Sauerstoff und Schwefel, welcher zusätzlich benzanelliert sein kann, tragende $C_1$- bis $C_{20}$-Alkylgruppen, wobei alle bei den Bedeutungen für R genannten Phenylkerne und heterocyclischen Ringe noch zusätzlich als Substituenten bis zu drei $C_1$- bis $C_4$-Alkylgruppen, Hydroxylgruppen, Carboxylgruppen, Sulfogruppen oder $C_1$- bis $C_4$-Alkoxycarbonylgruppen tragen können, steht, beispielsweise die Verbindungen α-Alanin-N,N-diacetonitril und α-Alaninnitril-N,N-diacetonitril, sowie verdoppelte Glycin-N,N-diacetonitrile und verdoppelte Glycinnitril-N,N-diacetonitrile der Formel

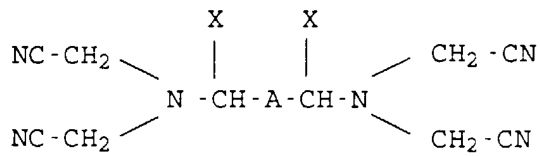

wobei X eine Carbonsäure- oder eine Nitril-Funktion bezeichnet, als Zwischenprodukte für die Herstellung von Glycin-N,N-diessigsäure-Derivaten I und ihren Salzen. Diese Verbindungen entstehen als Zwischenstufen bei der Umsetzung der genannten Glycin- bzw. Glycinnitril-Derivate mit Formaldehyd und Cyanwasserstoff bzw. von Iminodiacetonitril mit dementsprechenden Mono- oder Dialdehyden und Cyanwasserstoff.

Beim erfindungsgemäßen Verfahren läßt sich bei der "sauren" Variante bei Ausführungsform A mit in der 2-Position substituierten Glycinen oder verdoppelten Glycinen der Formel

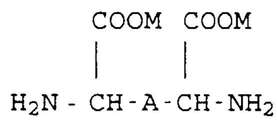

als Ausgangsmaterial auf zusätzliche Säure verzichten, da erstaunlicherweise die Acidität der vorhandenen Carboxylgruppe für die Durchführung der Umsetzung ausreichend ist.

Generell erhält man das Umsetzungsprodukt in hoher Ausbeute in ausreichend reiner Form. Der Gehalt an Nebenprodukten ist gering. Weitere Vorteile des erfindungsgemäßen Herstellungsverfahrens sind die salzfreie Fahrweise und die leicht verfügbaren Einsatzstoffe.

Gegenstand der vorliegenden Erfindung sind auch die in der Literatur noch nicht beschriebenen Glycin-N,N-diessigsäure-Derivate der allgemeinen Formel Ia

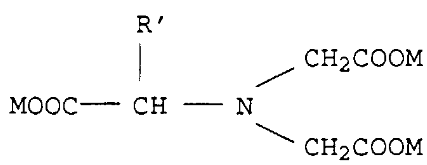

(Ia)

in der

R' für $C_4$- bis $C_{30}$-Alkyl, insbesondere $C_5$- bis $C_{30}$-Alkyl, oder $C_2$- bis $C_{30}$-Alkenyl, welche zusätzlich als Substituenten bis zu 5 Hydroxylgruppen, Formylgruppen, $C_1$- bis $C_4$-Alkoxygruppen, Phenoxygruppen oder $C_1$- bis $C_4$-Alkoxycarbonylgruppen tragen und durch bis zu 5 nicht benachbarte Sauerstoffatome unterbrochen sein können, Alkoxylat-Gruppierungen der Formel $-(CH_2)_k-O-(A^1O)_m-(A^2O)_n-Y$, in der $A^1$ und $A^2$ unabhängig voneinander 1,2-Alkylengruppen mit 2 bis 4 C-Atomen bezeichnen, Y Wasserstoff, $C_1$- bis $C_{12}$-Alkyl, Phenyl oder $C_1$- bis $C_4$-Alkoxycarbonyl bedeutet und k für die Zahl 1, 2 oder 3 sowie m und n jeweils für Zahlen von 0 bis 50 stehen, wobei die Summe aus m + n mindestens 4 betragen muß, Phenylalkylgruppen mit 1 bis 20 C-Atomen im Alkyl, einen fünf- oder sechsgliedrigen ungesättigten oder gesättigten heterocyclischen Ring mit bis zu drei Heteroatomen aus der Gruppe Stickstoff, Sauerstoff und Schwefel, welcher zusätzlich benzanelliert sein kann, tragende $C_1$- bis $C_{20}$-Alkylgruppen, wobei alle bei den Bedeutungen für R genannten Phenylkerne und heterocyclischen Ringe noch zusätzlich als Substituenten bis zu drei $C_1$- bis $C_4$-Alkylgruppen, Hydroxylgruppen, Carboxylgruppen, Sulfogruppen oder $C_1$- bis $C_4$-Alkoxycarbonylgruppen tragen können, oder einen Rest der Formel

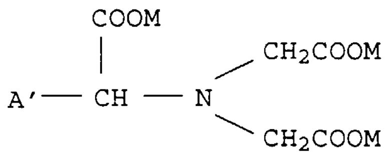

steht, wobei A' eine $C_1$- bis $C_{12}$-Alkylen-Brücke bezeichnet, und

M Wasserstoff, Alkalimetall, Erdalkalimetall-, Ammonium oder substituiertes Ammonium in den entsprechenden stöchiometrischen Mengen bedeutet.

Die Verbindungen I mit R = $C_1$- bis $C_3$-Alkyl sind bereits aus der Literaturstelle Chem. zvesti 28(3), 332-335 (1974) bekannt.

Herstellungsbeispiele

Beispiel 1

Herstellung von α-D,L-Alanin-N,N-diessigsäure-Trinatriumsalz aus Iminodiacetonitril

Zu einer Suspension von 95 g Iminodiacetonitril (100 gew.-%ig) in 500 ml Wasser wurden nacheinander bei 35 bis 50°C 14 g Schwefelsäure (100 gew.-%ig), 27 g wasserfreie Blausäure und 44 g Acetaldehyd (100 gew.-%ig) gegeben. Man rührte solange, bis bei der Titration des Blausäuregehaltes keine Änderung mehr festzustellen war. Nach Abkühlen auf 10°C wurde der Niederschlag abfiltriert und getrocknet. Es resultierten 123,4 g α-D,L-Alaninnitril-N,N-diacetonitril (entsprechend 83 % der Theorie) vom Schmelzpunkt 82°C.

Das erhaltene α-D,L-Alaninnitril-N,N-diacetonitril wurde bei 50°C in 440 g 25 gew.-%iger wäßriger Natronlauge eingetragen, es wurde anschließend noch 2 Stunden bei dieser Temperatur nachgerührt. Danach wurde für 10 Stunden auf 95°C erwärmt. Gegen Ende der Umsetzung wurde das Reaktionsgemisch mit Wasser verdünnt. Man erhielt so 610 g einer wäßrigen Lösung von α-D,L-Alanin-N,N-diessigsäure-Trinatriumsalz mit einem Eisen-Bindevermögen von 1,285 mmol/g (entsprechend 94 % der Theorie, bez. auf eingesetztes α-D,L-Alaninnitril-N,N-diacetonitril).

Beispiel 2

Herstellung von α-D,L-Alanin-N,N-diessigsäure-Trinatriumsalz aus α-D,L-Alanin

Zu einer Suspension von 44 g D,L-Alanin (> 99 gew.-%ig) in 200 g Wasser gab man bei 30°C gleichzeitig 105 g Formaldehyd (30 gew.-%ig) und 31,7 g Blausäure (89,5 gew.-%ig). Es wurde noch 3 Stunden bei 30°C nachgerührt. Die Blausäure-Abnahme entsprach einem Umsatz von > 97 % der Theorie.

Die so erhaltene wäßrige Lösung von α-D,L-Alanin-N,N-diacetonitril wurde bei 30°C zu 132 g 50 gew.-%iger Natronlauge getropft. Nach 8-stündigem Rühren bei 30°C wurde die Temperatur auf 95 bis 102°C erhöht. Nach weiteren 4 Stunden war die Umsetzung praktisch vollständig. Man erhielt 352,5 g einer Lösung, die gemäß ihrem Eisen-Bindevermögen 37,4 Gew.-% α-D,L-Alanin-N,N-diessigsäure-Trinatriumsalz enthielt (entsprechend einer Ausbeute von 97,4 % der Theorie über beide Stufen).

Beispiel 3

L-Tyrosin-N,N-diessigsäure-Trinatriumsalz aus L-Tyrosin

45,8 g Tyrosin wurden in 200 ml Wasser suspendiert und 7,5 g HCN (90 gew.-%ig) und 25 g Formaldehyd (30 Gew.-%) in wäßriger Lösung zugegeben. Nach 2,5 h bei 40°C war der Umsatz an Blausäure maximal und es wurden weitere 12,0 g HCN (90 gew.-%ig) und 40,0 g Formaldehyd (30 Gew.-%) in wäßriger Lösung bei pH 1 zugegeben. Nach weiteren 5 h bei 35°C und 4 h bei 80°C erhielt man eine Lösung von L-Tyrosin-N,N-diacetonitril in 94 % Ausbeute der Theorie.

Diese Lösung wurde bei 40°C zu 130 g 50 gew.-%iger wäßriger Natronlauge getropft. Nach 2 h bei 60°C und 2 h bei 95°C wurden 385 g einer Lösung von L-Tyrosin-N,N-diessigsäure-Trinatriumsalz mit einem Eisenbindevermögen von 0,543 mmol/g (entsprechend 89 % der theoretischen Ausbeute) erhalten.

Beispiel 4

D,L-Ethylglycin-N,N-diessigsäure-Trinatriumsalz aus Iminodiacetonitril

In eine Suspension von 570 g Iminodiacetonitril in 2070 g Wasser wurden nacheinander 41 g Schwefelsäure (96 gew.-%), 180 g Cyanwasserstoff (99 Gew.-%) und 385 g Propionaldehyd (99,5 Gew.-%) zugetropft und solange bei 35°C gerührt, bis durch Titration keine Änderung des Blausäuregehaltes mehr feststellbar war. Nach Abkühlen auf 10°C wurden 977 g (97 % Ausbeute der Theorie) D,L-Ethylglycinnitril-N,N-diacetonitril als Niederschlag durch Filtration mit einer Reinheit von 96,8 Gew.-% erhalten.

Der Niederschlag wurde dann in 4430 g einer 17 gew.-%igen wäßrigen Natronlauge bei 60°C eingetragen und 3 h bei 60°C und 10 h bei 95°C nachgerührt und am Ende mit Wasser verdünnt. Man erhielt dadurch 5275 g einer Lösung von D,L-Ethylglycon-N,N-diessigsäure-Trinatriumsalz mit einem Eisenbindevermögen von 0,985 mmol/g (entsprechend 89 % Ausbeute der Theorie).

Beispiel 5

D,L-Propylglycin-N,N-diessigsäure-Trinatriumsalz aus Iminodiacetonitril

In eine Suspension von 95 g Iminodiacetonitril in 550 g Wasser wurden nacheinander 14 g Schwefelsäure (96 Gew.-%), 26,9 g Cyanwasserstoff (99,3 %) und 79,3 g Butyraldehyd zugetropft und 4 h bei 35°C gerührt, bis durch Titration keine Änderung des Blausäuregehaltes mehr feststellbar war. Nach Abkühlen auf 10°C wurden 165 g (94 % Ausbeute der Theorie) D,L-n-Propylglycinnitril-N,N-diacetonitril durch Phasentrennung erhalten.

Von diesem Öl wurden dann 70,4 g in 350 g einer 15 gew.-%igen wäßrigen Natronlauge bei 40°C eingetragen und 2 h bei 95°C nachgerührt und anschließend mit Wasser verdünnt. Man erhielt dadurch 600 g einer Lösung von D,L-n-Propylglycin-N,N-diessigsäure-Trinatriumsalz mit einem Eisenbindevermögen von 0,573 mmol/g (entsprechend 86 % Ausbeute der Theorie).

Beispiel 6

D,L-1-Methylpropylglycin-N,N-diessigsäure-Trinatriumsalz aus Iminodiacetonitril

In eine Suspension von 95 g Iminodiacetonitril in 350 g Wasser wurden nacheinander 6 g Schwefelsäure (96 Gew.-%), 30 g Cyanwasserstoff (99,4 Gew.-%) und 103,2 g 2-Methylbutyraldehyd zugetropft und 2 h bei 35°C und 25 h bei 55°C gerührt, bis durch Titration keine Änderung des Blausäuregehaltes mehr feststellbar war. Nach Abkühlen auf 10°C wurden 167 g (88 % Ausbeute der Theorie) D,L-1-Methylpropylglycinnitril-N,N-diacetonitril durch Phasentrennung erhalten.

Von diesem Öl wurden dann 143 g in 600 g einer 18 gew.-%igen wäßrigen Natronlauge bei 40°C eingetragen und 20 h bei 95°h nachgerührt und anschließend mit Wasser verdünnt. Man erhielt dadurch 960 g einer Lösung von D,L-1-Methylpropylglycin-N,N-diessigsäure-Trinatriumsalz mit einem Eisenbindevermögen von 0,619 mmol/g (entsprechend 79 % Ausbeute der Theorie).

Beispiel 7

D,L-2-Methylpropylglycin-N,N-diessigsäure-Trinatriumsalz aus Iminodiacetonitril

In eine Suspension von 95 g Iminodiacetonitril in 350 g Wasser wurden nacheinander 7 g Schwefelsäure (96 Gew.-%), 30 g Cyanwasserstoff (98,3 Gew.-%) und 103,4 g 3-Methylbutyraldehyd zugetropft und 2 h bei 35°C und 3 h bei 50°C gerührt, bis durch Titration keine Änderung des Blausäuregehaltes mehr feststellbar war. Nach Abkühlen auf 10°C wurden 175 g (92 % Ausbeute der Theorie) D,L-2-Methylpropylglycinnitril-N,N-diacetonitril durch Phasentrennung erhalten.

Das erhaltene Öl wurde dann in 860 g einer 14 gew.-%igen wäßrigen Natronlauge bei 40°C eingetragen und 3 h bei 60°C und 5 h bei 95°C nachgerührt. Man erhielt dadurch 1070 g einer Lösung von D,L-2-Methylpropylglycin-N,N-diessigsäure-Trinatriumsalz mit einem Eisenbindevermögen von 0,775 mmol/g (entsprechend 90 % Ausbeute der Theorie).

Beispiel 8

D,L-n-Nonylglycin-N,N-diessigsäure aus Iminodiacetonitril

In eine Suspension von 95 g Iminodiacetonitril in 500 g Wasser wurden nacheinander 14 g Schwefelsäure (96 Gew.-%), 30,2 g Cyanwasserstoff (98,4 Gew.-%) und 172 g n-Decanal getropft und 17 h bei 60°C und 2 h bei 80°C gerührt, bis durch Titration keine Änderung des Blausäuregehaltes mehr feststellbar war. Nach Abkühlen auf 10°C wurde die Wasserphase abgetrennt und das verbleibende Öl zweimal mit 500 ml Wasser ausgeschüttelt, aus der organischen Phase wurden 205 g (79 % Ausbeute der Theorie) D,L-n-Nonylglycinnitril-N,N-diacetonitril erhalten.

Von diesem Öl wurden dann 205 g in 600 g einer 18 gew.-%igen wäßrigen Natronlauge zusammen mit 600 ml n-Butanol bei 40°C eingetragen und 30 h bei 95°C nachgerührt. Anschließend wurden die flüchtigen Anteile abdestilliert und der Rückstand in Wasser aufgenommen, mit HCl auf pH 1 gestellt und der sich bildende Niederschlag durch Filtration isoliert. Man erhielt dadurch 209 g D,L-n-Nonylglycin-N,N-diessigsäure mit einem Eisenbindevermögen von 2,57 mmol/g (entsprechend 68 % Ausbeute der Theorie).

Beispiel 9

D,L-n-Tridecylglycin-N,N-diessigsäure aus Iminodiacetonitril

In eine Suspension von 95 g Iminodiacetonitril in 500 g Wasser wurden nacheinander 14 g Schwefelsäure (96 Gew.-%), 30,2 g Cyanwasserstoff (98,4 Gew.-%) und 234 g n-Tetradecanal getropft und 17 h bei 60°C und 2 h bei 80°C gerührt, bis durch Titration keine Änderung des Blausäuregehaltes mehr feststellbar war. Nach Abkühlen auf 10°C wurde die Wasserphase abgetrennt und das verbleibende Öl zweimal mit 500 ml Wasser ausgeschüttelt, aus der organischen Phase wurden 259 g (82 % Ausbeute der Theorie) D,L-n-Tridecylglycinnitril-N,N-diacetonitril erhalten.

Von diesem Öl wurden dann 259 g in 600 g einer 18 gew.-%igen wäßrigen Natronlauge zusammen mit 600 ml n-Butanol bei 40°C eingetragen und 30 h bei 95°C nachgerührt. Anschließend wurden die flüchtigen Anteile abdestilliert und der Rückstand in Wasser aufgenommen, mit HCl auf pH 1 gestellt und der sich bildende wachsartige Niederschlag durch Filtration isoliert. Man erhielt dadurch 252 g D,L-n-Tetradecylglycin-N,N-diessigsäure mit einem Eisenbindevermögen von 2,14 mmol/g (entsprechend 66 % Ausbeute der Theorie).

Beispiel 10

D,L-(2-Phenylethylen)glycin-N,N-diessigsäure-Trinatriumsalz aus Iminodiacetonitril

In eine Suspension von 23,8 g Iminodiacetonitril in 125 g Methanol wurden nacheinander 3,5 g Schwefelsäure (96 Gew.-%), 8,0 g Cyanwasserstoff (98,3 Gew.-%) und 35,2 g 3-Phenylpropionaldehyd zugetropft und 50 h bei 50°C gerührt, nach dieser Zeit war der Umsatz nach Blausäuregehalt 95,5 % der Theorie.

Von der unbehandelten Lösung von D,L-(2-Phenylethylen)glycinnitril-N,N-diacetonitril in Methanol wurden dann 190 g in 186 g einer 19 gew.-%igen wäßrigen Natronlauge bei 40°C eingetragen und 3 h bei 60°C und weitere 22 h bei 95°C nachgerührt, wobei abdestillierendes Methanol durch Wasser ersetzt wurde. Man erhielt dadurch 510 g einer Lösung von D,L-(2-Phenylethylen)-glycin-N,N-diessigsäure-Trinatriumsalz mit einem Eisenbindevermögen von 0,368 mmol/g (entsprechend 75 % Ausbeute der Theorie). Durch Ansäuern auf pH 1,5, Absaugen des gebildeten Niederschlages und Waschen mit Methanol bei 40°C erhielt man die entsprechende freie Säure in 96 gew.-%iger Reinheit.

Beispiel 11

2-Furylmethylenglycin-N,N-diessigsäure aus Iminodiacetonitril

In eine Suspension von 47,5 g Iminodiacetonitril in 200 g Wasser wurden nacheinander 4,8 g Schwefelsäure (96 Gew.-%), 16,5 g Cyanwasserstoff (90,2 Gew.-%) und 52,9 g Furfural getropft und 6 h bei 60°C und 8 h bei 85°C gerührt, bis durch Titration keine Änderung des Blausäuregehaltes mehr feststellbar war. Die Mischung wurde mit Natriumchlorid gesättigt und dreimal mit Methyl-tert.-butylether ausgeschüttelt, die vereinigten organischen Phasen wurden auf -20°C gekühlt und der gebildete Niederschlag wurde isoliert. Es resultierten 95 g (89 % Ausbeute der Theorie) D,L-2-Furylmethylenglycinnitril-N-N-diacetonitril.

Von diesem Kristallisat wurden dann 46 g in 130 g einer 22 gew.-%igen wäßrigen Natronlauge bei 40°C eingetragen und 3 h bei 40°C und 4 h bei 95°C nachgerührt. Anschließend wurde mit HCl auf pH 1,5 gestellt und der sich bildende Niederschlag durch Filtration isoliert und mit Wasser gewaschen. Man erhielt dadurch 47 g D,L-2-Furylmethylenglycin-N,N-diessigsäure mit einem Eisen-bindevermögen von 3,61 mmol/g (entsprechend 79 % Ausbeute der Theorie).

Beispiel 12

1,3-Propylen-bis(D,L-glycin-N,N-diessigsäure) -Hexanatriumsalz aus Iminodiacetonitril

In eine Suspension von 95 g Iminodiacetonitril in 410 g Wasser wurden nacheinander 14 g Schwefelsäure (96 Gew.-%), 33,1 g Cyanwasserstoff (89,8 Gew.-%) und 220 g Glutardialdehyd (25 Gew.-% in Wasser) getropft und 2 h bei 35°C und 6 h bei 70°C gerührt, bis durch Titration keine Änderung des Blausäuregehaltes mehr feststellbar war (99,1 % Umsatz der Theorie). Nach Abkühlen auf 10°C wurde die Wasserphase abgetrennt und das verbleibende Öl zweimal mit 500 ml Wasser ausgeschüttelt, aus der organischen Phase wurden 149 g (97 % Ausbeute der Theorie) 1,3-Propylen-bis(D,L-glycinnitril-N,N-diacetonitril) erhalten.

Von diesem Öl wurden dann 149 g in 744 g einer 19 gew.-%igen wäßrigen Natronlauge bei 30°C eingetragen, 12 h bei 70°C und 11 h bei 100°C nachgerührt. Man erhielt 572 g einer Lösung von 1,3-Propylen-bis(D,L-glycin-N,N-diessigsäure)-Hexanatriumsalz mit einem Eisenbindevermögen von 0,829 mmol/g (entsprechend 99 % der theoretischen Ausbeute). Durch Zusatz von Methanol zu der Lösung gelang die Reinisolierung des Produktes.

Anwendungstechnische Daten und Anwendungsbeispiele

Bestimmung des Calcium-Bindevermögens

Meßprinzip

Die inhibierende Wirkung von Komplexbildnern oder Dispergiermitteln auf die Ausfällung von Calciumcarbonat wird durch Trübungstitration bestimmt. Es wird die zu untersuchende Substanz vorgelegt und in Gegenwart von Natriumcarbonat mit Calciumacetatlösung titriert. Der Endpunkt wird durch Bildung des Calciumcarbonat-Niederschlages angezeigt. Durch Verwendung einer ausreichenden Menge an Natriumcarbonat wird sichergestellt, daß die Messung auch dann ein korrektes Ergebnis liefert, wenn die Wirkung nicht nur auf einer Komplexierung der Calciumionen beruht, sondern auf der Dispergierung von Calciumcarbonat. Werden nämlich zu kleine Natriumcarbonatmengen eingesetzt, besteht die Gefahr, daß das Dispergiervermögen des Produktes nicht ausgeschöpft wird; in diesem Fall wird der Titrationsendpunkt durch die Fällung des Calcium-Salzes der untersuchten Verbindung bestimmt.

Während der Titration wird die Änderung der Lichtdurchlässigkeit mit Hilfe eines Lichtleiterphotometers verfolgt. Bei letzterem wird ein über Glasfaser in die Lösung geleiteter Lichtstrahl an einem Spiegel reflektiert und die Intensität des reflektierenden Lichts gemessen.

Reagenzien

0,25 m $Ca(OAC)_2$-Lösung
10 gew.-%ige $Na_2CO_3$-Lösung
1 n NaOH-Lösung
1 gew.-%ige Salzsäure

Durchführung

1 g Wirksubstanz (W.S.) in Form des Trinatriumsalzes wird in 100 ml destilliertem $H_2O$ gelöst. Anschließend werden 10 ml 10 gew.%ige $Na_2CO_3$-Lösung zugegeben. Bei Raumtemperatur (RT) und einem während der Titration konstant gehaltenen pH-Wert von 11 und bei 80°C mit einem pH-Wert von 10 wird mit 0,25 m $Ca(OAc)_2$-Lösung kontinuierlich mit 0,2 ml/min automatisch titriert.

Berechnung

Menge mg $CaCO_3$/g W.S. = Verbrauch an $Ca(OAc)_2$-Lösung in ml x 25. Bei der automatischen Titration ist der 1. Knickpunkt der Titrationskurve der Endpunkt.

Weiterhin wurde die Perborat-Stabilisierung der Reinigungsmittelformulierungen 1 und 2 der unten bezeichneten Zusammensetzungen bestimmt

Das für die Bleichwirkung in natriumperborathaltigen Waschmittelformulierungen verantwortliche Wasserstoffperoxid wird durch Schwermetallionen (Fe, Cu, Mn) katalytisch zersetzt. Durch Komplexierung der Schwermetallionen läßt sich dies verhindern. Die peroxidstabilisierende Wirkung der Komplexbildner wird über den Restperoxidgehalt nach Warmlagerung einer schwermetallhaltigen Waschflotte geprüft.
Der Gehalt an Wasserstoffperoxid wurde vor und nach der Lagerung durch eine Titration mit Kaliumpermanganat in saurer Lösung bestimmt.

Zur Prüfung auf Perboratstabilisierung werden zwei Waschmittelformulierungen benutzt, wobei die Zersetzung bei der Warmlagerung durch Zugabe von Schwermetallkatalysatoren (2,5 ppm Mischung aus 2 ppm $Fe^{3+}$, 0,25 ppm $Cu^{2+}$, 0,25 ppm $Mn^{2+}$) erfolgt.

1. Phosphathaltige Formulierung

| Zusammensetzung (in Gew.-%): | |
|---|---|
| 19,3 % | Natrium-$C_{12}$-Alkylbenzolsulfonat (50 gew.-%ige wäßrige Lösung) |
| 15,4 % | Natriumperborat ·4 $H_2O$ |
| 30,8 % | Natriumtriphosphat |
| 2,6 % | Copolymer aus Maleinsäure und Acrylsäure (Gew.-Verhältnis 50:50, mittleres Molekulargewicht 50.000) |
| 31,0 % | Natriumsulfat, wasserfrei |
| 0,9 % | erfindungsgemäßer Komplexbildner oder Vergleichsverbindung |

Die Waschmittelkonzentration betrug 6,5 g/l unter Verwendung von Wasser mit 25°dH. Die Lagerung erfolgte bei 80°C 2 Stunden lang.

2. Phosphatreduzierte Formulierung

| Zusammensetzung (in Gew.-%): | |
|---|---|
| 15 % | Natrium-$C_{12}$-Alkylbenzolsulfonat (50 gew.-%ige wäßrige Lösung) |
| 5 % | Anlagerungsprodukt von 11 Mol Ethylenoxid an 1 Mol Talgfettalkohol |
| 20 % | Natriumperborat · 4 $H_2O$ |
| 6 % | Natrium-metasilikat · 5 $H_2O$ |
| 1,25 % | Magnesiumsilikat |
| 20 % | Natriumtriphosphat |
| 31,75 % | Natriumsulfat, wasserfrei |
| 1 % | erfindungsgemäßer Komplexbildner oder Vergleichsverbindung |

Die Waschmittelkonzentration betrug 8 g/l unter Verwendung von Wasser mit 25°dH. Die Lagerung erfolgte bei 60°C 1 Stunde lang.

Die folgende Tabelle 1 zeigt die Ergebnisse der Bestimmungen.

Tabelle 1

| Komplex-bildner | Calciumcarbonat-Dispergier-Kapazität mg | | | | Perborat-Stabilisierung [%] Formulierung | |
|---|---|---|---|---|---|---|
| | $CaCO_3$/g RT pH 11 | W.S. 80°C pH 10 | $CaCO_3$/mol RT pH 11 | W.S. 80°C pH 10 | 1 | 2 |
| | | | | | | |
| α-ADA-$Na_3$ aus Bsp. Nr. 2 | 370 | 330 | 1,00 | 0,89 | 32,4 | 40,8 |

α-ADA-$Na_3$ = α-Alanin-N,N-diessigsäure-Trinatriumsalz

Tabelle 1 (fortgesetzt)

| Komplex-bildner | Calciumcarbonat-Dispergier-Kapazität mg | | | | Perborat-Stabilisierung [%] Formulierung | |
|---|---|---|---|---|---|---|
| zum Vergleich: | | | | | | |
| Penta-natrium triphosphat | 215 | 150 | 0,79 | 0,55 | - | - |
| $NTA-Na_3$ | 350 | 250 | 0,90 | 0,64 | 24,5 | 32,5 |
| $EDTA-Na_4$ | 275 | 240 | 1,04 | 0,91 | 20,0 | 34,0 |

$NTA-Na_3$ = Nitrilotriessigsäure-Trinatriumsalz
$EDTA-Na_4$ = Ethylendiamintetraessigsäure-Tetranatriumsalz

Bestimmung des Mangan-Bindevermögens

Meßvorschrift

10,0 ml 0,005 m $MnSO_4 \cdot H_2O$-Lösung werden mit 50 ml destilliertem Wasser, 10 Tropfen 5 gew.-%iger Kaliumnatriumtartratlösung, ca. 3 ml einer Pufferlösung, ca. 30 mg Ascorbinsäure und einer Spatelspitze Indikator (1 Gew.-Teil Eriochromschwarz T mit 400 Gew.-Teilen NaCl verrieben) versetzt und auf 75°C erwärmt. Diese Lösung wird mit einer 0,001 m Lösung des Komplexbildners (K.B.) bis zum bleibenden Umschlag nach blau titriert.

Auswertung

$$\text{mg Mn}^{2+}\text{/g K.B.} = \frac{274{,}7 \times 1000}{\text{verbrauchte ml} \times \text{Molgewicht K.B.}}$$

$$\text{mol Mn}^{2+}\text{/mol K.B.} = \frac{274{,}7 \times 1000}{\text{verbrauchte ml} \times 54{,}94}$$

Die folgende Tabelle 2 zeigt die Ergebnisse der Bestimmungen.

Tabelle 2

| Komplexbildner | mg $Mn^{2+}$/g Komplexbildner | mol $Mn^{2+}$/mol Komplexbildner |
|---|---|---|
| $\alpha$-ADA-$Na_3$ aus Bsp. Nr. 2 | 209 | 0,86 |
| zum Vergleich: $EDTA-Na_4$ | 192 | 1,02 |

Beispiel 13

Hochalkalische Reinigungsmittelformulierung für Molkereien

| Eine Mischung aus | |
|---|---|
| 40 Gew.-Teilen | 50 gew.-%iger Natronlauge, |
| 20 Gew.-Teilen | einer 30 gew.-%igen wäßrigen Lösung von $\alpha$-D,L-Alanin-N,N-diessigsäure-Trinatriumsalz aus Bsp. Nr. 2, |
| 4 Gew.-Teilen | eines $C_{10}$-Oxoalkoholethoxylates mit einem Ethoxylierungsgrad von ca. 4, |
| 4 Gew.-Teilen | einer handelsüblichen Alkylcarbonsäure-Mischung als Lösevermittler, |
| 7 Gew.-Teilen | Natriumgluconat zum Abbau der Wasserhärte und |
| 25 Gew.-Teilen | Wasser |

wurde bei der Entfernung von Ablagerungen aus Calciumphosphat, Calciumoxalat, Eiweiß und Asche eingesetzt. Die Ablagerungen ließen sich problemlos entfernen.

Beispiel 14

Hochalkalische Reinigungsmittelformulierung für Brauereien

Eine Mischung aus

| | |
|---|---|
| 40 Gew.-Teilen | 50 gew.-%iger Kalilauge, |
| 20 Gew.-Teilen | einer 30 gew.-%igen wäßrigen Lösung von α-D,L-Alanin-N,N-diessigsäure-Trinatriumsalz aus Bsp. Nr. 2, |
| 3 Gew.-Teilen | eines $C_{10}$-Oxoalkoholethoxylates mit einem Ethoxylierungsgrad von ca. 3, |
| 3 Gew.-Teilen | einer handelsüblichen Alkylcarbonsäure-Mischung als Lösevermittler und |
| 34 Gew.-Teilen | Wasser |

wurde bei der Entfernung von Ablagerungen aus Calciumoxalat, Hopfenharzen und Eiweiß eingesetzt. Die Ablagerungen ließen sich problemlos entfernen.

## Patentansprüche

**1.** Verwendung von Glycin-N,N-diessigsäure-Derivaten der allgemeinen Formel I

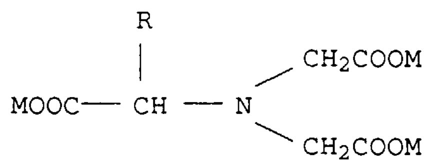

(I)

in der

R für $C_1$- bis $C_{30}$-Alkyl oder $C_2$- bis $C_{30}$-Alkenyl, welche zusätzlich als Substituenten bis zu 5 Hydroxylgruppen, Formylgruppen, $C_1$- bis $C_4$-Alkoxygruppen, Phenoxygruppen oder $C_1$- bis $C_4$-Alkoxycarbonylgruppen tragen und durch bis zu 5 nicht benachbarte Sauerstoffatome unterbrochen sein können, Alkoxylat-Gruppierungen der Formel $-(CH_2)_k—O—(A^1O)_m—(A^2O)_n—Y$, in der $A^1$ und $A^2$ unabhängig voneinander 1,2-Alkylengruppen mit 2 bis 4 C-Atomen bezeichnen, Y Wasserstoff, $C_1$- bis $C_{12}$-Alkyl, Phenyl oder $C_1$- bis $C_4$-Alkoxycarbonyl bedeutet und k für die Zahl 1, 2 oder 3 sowie m und n jeweils für Zahlen von 0 bis 50 stehen, wobei die Summe aus m + n mindestens 4 betragen muß, Phenylalkylgruppen mit 1 bis 20 C-Atomen im Alkyl, einen fünf- oder sechsgliedrigen ungesättigten oder gesättigten heterocyclischen Ring mit bis zu drei Heteroatomen aus der Gruppe Stickstoff, Sauerstoff und Schwefel, welcher zusätzlich benzanelliert sein kann, tragende $C_1$- bis $C_{20}$-Alkylgruppen, wobei alle bei den Bedeutungen für R genannten Phenylkerne und heterocyclischen Ringe noch zusätzlich als Substituenten bis zu drei $C_1$- bis $C_4$-Alkylgruppen, Hydroxylgruppen, Carboxylgruppen, Sulfogruppen oder $C_1$- bis $C_4$-Alkoxycarbonylgruppen tragen können, oder einen Rest der Formel

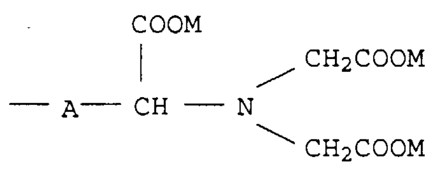

steht, wobei A eine $C_1$- bis $C_{12}$-Alkylen-Brücke, vorzugsweise eine $C_2$- bis $C_{12}$-Alkylen-Brücke, oder eine chemische Bindung bezeichnet, und

M Wasserstoff, Alkalimetall, Erdalkalimetall, Ammonium oder substituiertes Ammonium in den entsprechenden stöchiometrischen Mengen bedeutet,

als Komplexbildner für Erdalkali- und Schwermetallionen in pulverförmigen Kompakt- und Ultra-Kompakt-Waschmittelformulierungen und in flüssigen Waschmittelformulierungen.

**2.** Pulverförmige Kompakt- und Ultra-Kompakt-Waschmittelformulierungen mit einer Schüttdichte von ≥ 700 g/l, enthaltend ein oder mehrere Glycin-N,N-diessigsäure-Derivate I gemäß Anspruch 1.

**3.** Pulverförmige Kompakt- und Ultra-Kompakt-Waschmittelformulierungen nach Anspruch 2, enthaltend die Glycin-N,N-diessigsäure-Derivate I in einer Menge von 0,1 bis 25 Gew.-%.

**4.** Flüssige Waschmittelformulierungen, enthaltend ein oder mehrere Glycin-N,N-diessigsäure-Derivate I gemäß Anspruch 1.

**5.** Flüssige Waschmittelformulierungen nach Anspruch 4, enthaltend die Glycin-N,N-diessigsäure-Derivate I in einer Menge von 0,05 bis 1 Gew.-%.

Europäisches Patentamt

# EUROPÄISCHER RECHERCHENBERICHT

Nummer der Anmeldung
EP 99111836.5

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁶) |
| A | CHEMICAL ABSTRACTS, Band 58, Nr. 5, 04. März 1963 Columbus, Ohio, US, Zusammenfassungsnr. 4644e, KAZARINOVA, N.F. ET AL.: "Investigation of complexing agents derived from amino acids"; & Tr. Soveshch. po Kompleksonam, Akad. Nauk SSSR, Ural'sk. Filial, Sverdlovsk 1958, 39-52, Zusammenfassung. -- | 1-5 | C11D3/33<br>C11D17/00<br>C11D17/06<br>C07C229/16 |
| A | CHEMICAL ABSTRACTS, Band 103, Nr. 14, 07. Oktober 1985 Columbus, Ohio, US, Zusammenfassungsnr. 110849m, LUBKEOVA, S. ET AL.: "New complexanes. VL. Acid-base chelate-forming properties of surface-active complexanes of the type of 2-alkylnitrilotriacetic acids" Seite 396, Spalte 2; & Chem. Pap. 1985, 39(3), 317-27 (Eng), Zusammenfassung. -- | 1-5 | RECHERCHIERTE SACHGEBIETE (Int. Cl.⁶)<br>C11D<br>C07C229/00 |
| A | DE 4211713 A (BASF AG) 14. Oktober 1993, Seite 3, Zeile 41 - Seite 4, Zeile 34, Anspruch 1. -- | 1-5 | |
| A | DE 1643415 A (HOOKER CHEMICAL CORP.) 03. Juni 1971, Ansprüche. -- | 1-5 | |
| A | GB 2178754 A | 1-5 | |

Der vorliegende Recherchenbericht wurde für alle Patentansprüche erstellt.

| Recherchenort | Abschlußdatum der Recherche | Prüfer |
|---|---|---|
| WIEN | 09-08-1999 | SEIRAFI |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

EP 99111836.5

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl.⁴) |
| | (COLGATE-PALMOLIVE COMPANY) 18. Februar 1987, Beispiele, Ansprüche 1,18. -- | | |
| A | DE 3712329 A (BASF AG) 20. Oktober 1988, Beispiele, Ansprüche. ---- | 1-5 | |
| | | | RECHERCHIERTE SACHGEBIETE (Int. Cl.⁴) |

Der vorliegende Recherchenbericht wurde für alle Patentansprüche erstellt.

| Recherchenort | Abschlußdatum der Recherche | Prüfer |
|---|---|---|
| WIEN | 09-08-1999 | SEIRAFI |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

ANHANG ZUM EUROPÄISCHEN RECHERCHENBERICHT
ÜBER DIE EUROPÄISCHE PATENTANMELDUNG NR. EP 99111836.5

In diesem Anhang sind die Mitglieder der Patentfamilien der im obengenannten europäischen Recherchenbericht angeführten Patentdokumente angegeben.
Die Angaben über die Familienmitglieder entsprechen dem Stand der EPIDOS-INPADOC-Datei am 27.10.1999
Diese Angaben dienen zur Unterrichtung und erfolgen ohne Gewähr.

| Im Recherchenbericht angeführtes Patentdokument | | Datum der Veröffentlichung | Mitglied(er) der Patentfamilie | | Datum der Veröffentlichung |
|---|---|---|---|---|---|
| DE A1 | 4211713 | 14-10-1993 | keine | | |
| DE A1 | 1642415 | | BE A | 705235 | 01-03-1968 |
| | | | CH A | 530961 | 15-01-1973 |
| | | | DE A | 1643415 | 03-06-1971 |
| | | | GB A | 1184608 | 18-03-1970 |
| | | | NL A | 6713990 | 22-04-1968 |
| | | | SE B | 354651 | 19-03-1973 |
| | | | SE C | 354651 | 13-03-1975 |
| | | | US A | 3733355 | 15-05-1973 |
| GB A1 | 2178754 | 18-02-1987 | US A | 4769168 | 06-09-1988 |
| | | | AT A | 2054/86 | 15-07-1993 |
| | | | AU A1 | 60738/86 | 12-02-1987 |
| | | | AU B2 | 590294 | 23-11-1989 |
| | | | BE A1 | 905217 | 04-02-1987 |
| | | | BR A | 8603676 | 10-03-1987 |
| | | | CA A1 | 1293903 | 07-01-1992 |
| | | | CH A | 671234 | 15-08-1989 |
| | | | DE A1 | 3625139 | 12-02-1987 |
| | | | DK A0 | 3732/86 | 05-08-1986 |
| | | | DK A | 3732/86 | 06-02-1987 |
| | | | DK B1 | 166763 | 12-07-1993 |
| | | | EG A | 18138 | 30-08-1993 |
| | | | ES AF | 2000933 | 16-03-1988 |
| | | | FR A1 | 2585721 | 06-02-1987 |
| | | | GB B2 | 2178754 | 06-09-1989 |
| | | | GR A | 862054 | 04-03-1987 |
| | | | HK A | 384/92 | 18-09-1992 |
| | | | IL A0 | 79940 | 31-12-1986 |
| | | | IL A1 | 79940 | 05-11-1990 |
| | | | IN A | 166259 | 31-03-1990 |
| | | | IT A0 | 8648360 | 05-08-1986 |
| | | | IT A | 1214710 | 18-01-1990 |
| | | | JP A2 | 62049795 | 03-03-1987 |
| | | | KR B1 | 9410117 | 21-10-1994 |
| | | | LU A | 86544 | 06-03-1987 |
| | | | MX B | 164112 | 17-07-1992 |
| | | | NL A | 8601996 | 02-03-1987 |
| | | | NO A0 | 863143 | 04-08-1986 |
| | | | NO A | 863143 | 04-02-1987 |
| | | | NO B | 169239 | 17-02-1992 |
| | | | NO C | 169239 | 27-05-1992 |
| | | | NZ A | 216994 | 28-06-1989 |
| | | | PH A | 23487 | 16-08-1989 |
| | | | PT A | 83121 | 01-09-1986 |
| | | | PT B | 83121 | 29-07-1988 |
| | | | SE A0 | 8603265 | 31-07-1986 |
| | | | SE A | 8603265 | 06-02-1987 |
| | | | SE B | 468395 | 11-01-1993 |
| | | | SE C | 468395 | 06-05-1993 |
| | | | SG A | 725/92 | 02-10-1992 |
| | | | ZW A | 150/86 | 16-09-1987 |
| | | | AR A1 | 240837 | 28-02-1991 |
| | | | GB A0 | 8618057 | 10-09-1986 |
| | | | TR A | 25331 | 01-01-1993 |
| | | | US A | 4690774 | 01-09-1987 |
| | | | ZA A | 8605635 | 30-03-1988 |
| | | | ZM A | 67/86 | 29-04-1985 |
| DE A1 | 3712329 | 20-10-1988 | AT E | 72229 | 15-02-1992 |
| | | | AU A1 | 14463/88 | 13-10-1988 |
| | | | AU B2 | 608592 | 11-04-1991 |
| | | | CA A1 | 1313673 | 16-02-1993 |
| | | | DE C0 | 3868110 | 12-03-1992 |
| | | | EP A1 | 287885 | 26-10-1988 |
| | | | EP B1 | 287885 | 29-01-1992 |
| | | | ES T3 | 2028924 | 16-07-1992 |
| | | | JP A2 | 63267751 | 04-11-1988 |
| | | | US A | 4973730 | 27-11-1990 |
| | | | US A | 5019296 | 28-05-1991 |

Bezüglich näherer Einzelheiten zu diesem Anhang siehe Amtsblatt des Europäischen Patentamtes, Nr. 12/82.